# EUROPEAN PATENT SPECIFICATION

(11) **EP 2 234 955 B1**
(45) Date of publication and mention of the grant of the patent: **10.08.2011**
(21) Application number: 08870647.8
(22) Date of filing: 29.12.2008
(51) Int. Cl.: C07C 67/08, C07C 67/60, C07C 69/54, C07C 303/44

(54) **PROCESS FOR RECOVERING SULFONIC ACID CATALYST AND NOBLE PRODUCTS FROM ACRYLATE HEAVY ENDS**
VERFAHREN ZUR RÜCKGEWINNUNG EINES SULFONSÄUREKATALYSATORS UND WERTPRODUKTEN AUS ACRYLATNACHLAUFFRAKTIONEN
PROCÉDÉ DE RÉCUPÉRATION D'UN CATALYSEUR ACIDE SULFONIQUE ET DE PRODUITS NOBLES À PARTIR DE FRACTIONS LOURDES D'ACRYLATE

(30) Priority: 18.01.2008 US 22034
(43) Date of publication of application: 06.10.2010
(73) Proprietor: Arkema, Inc., Philadelphia, Pennsylvania 19103 (US)
(72) Inventor: JAWAID, Mahmood, N.A., Cros Lanes, WV 25313 (US); FRUCHEY, Olan, S., Hurricane WV 25526 (US); BROOKS, William, C., St. Albans WV 25177 (US); HO, Fungau, Charleston WV 25314 (US)
(74) Representative: Bonnel, Claudine
(86) International application number: PCT/US2008/088447
(87) International publication number: WO 2009/091491

(56) References cited:
- EP-A- 0 779 268
- US-A- 5 386 052
- US-B1- 6 172 258

## Description

### FIELD OF THE INVENTION

This invention relates to a process of making alkyl esters of (meth)acrylic acid. In one aspect, the invention relates to the process in which a strong sulfonic acid catalyst is employed while in another aspect, the invention relates to the process in which the strong sulfonic acid catalyst is eventually recovered and recycled. In still another aspect, the invention relates to the process in which the strong sulfonic acid catalyst along with noble products, is recovered from the acrylate heavy ends of the process.

### BACKGROUND OF THE INVENTION

Processes for preparing alkyl esters of (meth)acrylic acid by the reaction of a (meth)acrylic acid with a C₁₋₈ alkanol in the presence of a strong sulfonic acid catalyst, e.g., methane sulfonic acid (MSA), in a homogeneous liquid phase at an elevated temperature are well known. See, for example, USP 6,172,258 and 6,472,554. In these processes, equimolar amounts of (meth)acrylic acid and alcohol are fed to a reactor, preferably to the base of a distillation column, with an acid catalyst and subjected to esterification conditions. The bulk of the (meth)acrylate ester and unreacted alcohol are distilled overhead, preferably using water as an azeotroping agent. The overhead vapors consists mainly of (meth)acrylate ester, a small amount of unreacted alcohol, water and minor amounts of light ends, i.e., reaction products having a boiling point lower than the (meth)acrylate ester. This overhead vapor is condensed and separated into two phases. The organic phase is subjected to purification, and the aqueous phase is returned to the column. The residue from the reaction vessel, i.e., the base of the distillation column, comprises mainly low molecular weight polymers of (meth)acrylic acid and (meth)acrylate ester, some free (meth)acrylic acid, alcohol and catalyst, and this residue is recovered and subjected to a heat treatment to crack the polymers. Most of the heat treated residue is returned to the reaction vessel, but a slip stream is sent to a heavy ends removal unit, e.g., a distillation column, in which the volatiles are removed overhead and recycled to the reactor while the residue from this heavy ends removal unit are discarded in an environmentally acceptable manner (unless subjected to still further treatment).

In another embodiment of the process, e.g., such as that taught in USP 5,386,052, only the water of reaction is removed from the reaction mixture. The product mixture comprises unreacted starting materials, principal products (e.g., (meth)acrylate esters), by-products (e.g., oligomers and polymers of the (meth)acrylate esters, water, etc.), catalyst and process aids (e.g., an azeotropic agent, a polymerization inhibitor, etc.). The product mixture is typically cooled and then washed with water in an extraction column to remove the catalyst and (meth)acrylic acid as a bottom stream, and the remainder of the product mixture as an overhead stream. This overhead stream can be further washed with water or an aqueous alkaline solution to further remove the remaining catalyst and acid, and this second washed stream can then be distilled to recover the principal product. This principal product is typically recovered as an overhead stream, and the remainder of the product mixture, i.e., the heavy ends, is recovered as a bottoms stream. Some of the unreacted starting materials and principal product still remaining in these heavy ends can be recovered by evaporation, but that which is not recovered and the other components of the heavy ends are lost unless subjected to still further treatment.

One such further treatment is described in USP 5,734,075. The by-product component of the heavy ends, i.e., the oligomers and polymers of the (meth)acrylates and (meth)acrylic acid, is subjected to cracking in the absence of a catalyst. Since catalyst is not present is this process, it cannot be recovered. The temperature range of the cracking is between 140-260°C. The light traction originating from the dissociation reactions is vaporized continuously during the cracking operation so as to recover the noble products, i.e., (meth)acrylic acid monomer, (meth)acrylate monomer and alcohol.

USP 5,877,345 and 6,180,819 teach a process for treating heavy ends produced during the production of an alkyl acrylate, the process comprising the steps of (a) feeding a total aqueous and heavy end feed stream comprising the heavy ends, water and residual acid catalyst to a hydrolysis reactor, and (b) distilling an overhead stream containing acrylic acid, alkyl acrylate, alkanol and water. The overhead stream is condensed, separating the overhead stream into an organic phase of noble products and an aqueous phase that is recycled to the hydrolysis reactor. Preferably, the hydrolysis reactor is used in conjunction with a cracking reactor into which a bleed stream from the hydrolysis reactor is fed and cracked to produced and recover acrylic acid, acrylate ester, alkanol and water. However, this process does not recover the sulfonic acid after hydrolysis.

USP 6,084,122 describes a process for the removal of sulfur from an acrylate waste stream. The process comprises (a) forming in a reactor a reaction mixture that contains an ester reaction product and residue byproducts, (b) recovering the reaction product by distillation, (c) transferring the residue of the reaction mixture to an evaporator for the recovery oil residual ester product and reactants, (d) contacting the residue of the evaporator in an agitated Liquid-liquid counter-current extraction column with water to form a two-phase system of (1) acid catalyst and water, and (2) heavy ends and oligomers, and (e) recycling the aqueous phase to the reactor. The heavy ends and oligomers are transferred for further handling, not described, or for disposal.

USP 6,084,128 describes a process in which an esterification product mixture containing a sulfonic acid catalyst is directed to a decanter or extractor and allowed to form a two-phase system of (i) catalyst and water, and (ii) reaction product, acrylate ester, solvent, heavy ends and oligomers. The catalyst and water phase is then recycled back to the reactor. Treatment of the heavy ends is not discussed

The product mixture from the esterification zone of the process described in USP 6,472,554, referenced above, is subjected to a three-stage pre-purification followed by rectification for isolating the ester product. In the first stage of the pre-purification, most of the esterification catalyst and high boilers are separated by washing, and are then recycled to the esterification zone. To control the build-up of high boilers, a part-stream of the recycle stream is removed from circulation. In the second stage, the strongly acidic components of the remaining reaction mixture are neutralized and extracted with an aqueous alkali solution. In the third stage, residual salts and aqueous foreign-phase fractions are removed by extraction with water from the organic reaction mixture remaining after the second pre-purification stage. The purification stage proper is conventional distillation.

USP 6,512,138 describes an esterification process in which unconverted starting compounds and (meth)acrylate products are separated off by distillation, and an oxyester-containing bottom product is formed and separated off. The oxyesters of the bottom product are cleaved in the presence of an acid catalyst and the cleavage products removed leaving a cleavage residue. Alternatively, the oxyesters are first removed from the cleavage product by distillation leaving a distillation residue. The removed oxyesters are then cleaved in the presence of an acid catalyst, and the cleavage products are recovered leaving a cleavage residue. The cleavage residue of either alternative is combined with the distillation residue and hydrolytically cleaved in the presence of water and acids or bases. The cleavage reaction can occur in a heatable, stirred reactor or a forced-circulation evaporator. Again the sulfonic acid is not recovered.

(Meth)acrylate polymers, or polymeric (meth)acrylates, are formed by free radical polymerization of the (meth)acrylates. These polymers form when the (meth)acrylates are subjected to heat, and they cannot be cleaved back into the starting monomers. (Meth)acrylate oligomers, or oligomeric (meth)acrylates, are the Michael adducts of (meth)acrylate with (meth)acrylic acid or alcohol. Oligomeric (meth)acrylates can be cleaved back into its starting monomers.

### SUMMARY OF THE INTENTION

Sulfonic acid and noble products contained in (meth)acrylate heavy ends, the heavy ends comprising the sulfonic acid, noble products and Michael adducts of the sulfonic acid and (meth)acrylate esters, are recovered by a process comprising the steps of (i) mixing the heavy ends with water, (ii) subjecting the mixture to sufficient heat and distillation conditions to crock and/or hydrolyze the Michael adducts into its constituent sulfonic acid and acrylate ester components, and (iii) recovering the sulfonic acid in an aqueous solvent extraction stream, and the noble products as a distillate stream. The Michael adducts are hydrolyzed to a hydroxy acid and ester which are further dehydrated (i.e., cracked) to (meth)acrylic acid monomer and (meth)acrylate ester monomer.

In one embodiment, the heavy ends are mixed with about an equal volume of water, and the mixture is agitated during the simultaneous cracking/hydrolysis and distillation operations. The mixture contains alcohol, e.g., butanol, and a (meth)acrylate ester, e.g., butyl acrylate, which acts as an azeotropic agent. The distillate is in two phases, and the aqueous phase is continuously returned to the distillation unit. (Meth)acrylate, alcohol and (meth)acrylic acid are recovered as an overhead organic distillate stream. The sulfonic acid is recovered from the aqueous layer in the distillation unit after the distillation is complete, In another embodiment, the cracking/hydrolysis and distillation results in an increase in sulfonic acid recovery from 90-94% to 130-145%% (measured by titration), an increase in nobles product recovery of 20-33%, and a reduction in waste, e.g., lost catalyst, starting material and/or product, of 24 to 35%. The recovered sulfonic acid can be recycled to the reactor to provide catalyst recycle which results in cost savings. Recycle of the noble product distillate stream to the reactor also increases starting material efficiency.

The greater than 100% recovery of sulfonic acid catalyst is an artifact of the calculation method. The sulfonic acid is present in the heavy ends in two forms, i.e., free sulfonic acid and bound sulfonic acid. The free sulfonic acid can be titrated with base, but the bound sulfonic acid cannot be titrated by base. The bound sulfonic acid is present as a Michael adduct with the acrylate ester. The Michael adduct is no longer acidic, and it cannot be titrated by base. However, upon hydrolysis/cracking the bound sulfonic acid is converted into free sulfonic acid which can be titrated with base. This yields recovery values greater than 100% based on titration.

in one embodiment, the invention is a process for producing a (meth)acrylate ester, the process comprising the steps of:
A. Subjecting a reaction mixture comprising (meth)acrylic acid, an alcohol and a sulfonic acid catalyst to esterification conditions so as to form a product mixture comprising unreacted (meth)acrylic acid and alcohol, sulfonic acid catalyst, (meth)acrylate ester, and oligomeric and polymeric (meth)acrylate esters;
B. Distilling the product mixture to recover (meth)acrylate ester, and to leave a distillation residue comprising sulfonic acid catalyst and oligomeric and polymeric acrylate esters;
C. Mixing the distillation residue with water to form a mixture of distillation residue and water;
D. Subjecting the distillation residue and water mixture of (C) to conditions such that, simultaneously, (1) oligomeric and polymeric (meth)acrylate esters are hydrolyzed and/or cracked to (meth)acrylate ester, (meth)acrylic acid, alcohol and sulfonic acid, (2) (meth)acrylic acid, alcohol and (meth)acrylate ester are recovered as an overhead distillation product, and (3) sulfonic acid catalyst is recovered by aqueous phase separation of the distillation residue and water mixture as a bottom product; and
E. Recycling at least a part of the overhead distillation and the aqueous bottom products of (D) to the reaction mixture of (A).
The return of the overhead distillation and aqueous bottom products to the reaction mixture of (A) provides catalyst recycle and an increase in the use of the starting materials.

### DESCRIPTION OF THE PREFERRED EMBODIMENT

All references to the Periodic Table of the Elements refer to the Periodic Table of the Elements, publishes and copyrighted by CRC Press, Inc., 2003. Also, any references to a Group or Groups shall be to the Group or Groups reflected in this Periodic Table of the Elements using the IUPAC system for numbering groups. Unless stated to the contrary, implicit from the context, or customary in the art, all parts and percents are based on weight and all test methods are current as of the filing date of this disclosure.

The numerical ranges in this disclosure are approximate, and thus may include values outside of the range unless otherwise indicated. Numerical ranges include all values from and including the lower and the upper values, in increments of one unit, provided that there is a separation of at least two units between any lower value and any higher value. As an example, if a compositional, physical or other property, such as, for example, molecular weight, viscosity, melt index, etc., is from 100 to 1,000, it is intended that all individual values, such as 100, 101, 102, *etc*., and sub ranges, such as 100 to 144, 155 to 170, 197 to 200, etc., are expressly enumerated. For ranges containing values which are less than one or containing fractional numbers grater than one (e.g., 1.1, 1,5, etc.), one unit is considered to be 0.0001, 0.001, 0.01 or 0.1, as appropriate. For ranges containing single digit numbers less than ten (e.g., 1 to 5), one unit is typically considered to be 0.1. These are only examples of what is specifically intended, and all possible combinations of numerical values between the lowest value and the highest value enumerated, are to be considered to be expressly stated in this disclosure. Numerical ranges are provided within this disclosure for, among other things, the relative amount of reagents and catalyst in the reaction mixture or mass, and various temperature and other process parameters.

The term "comprising" and its derivatives are not intended to exclude the presence of any additional component step or procedure, whether or not the same is specifically disclosed. In order to avoid any doubt, all compositions claimed through use of the term "comprising" may include any additional additive, adjuvant, or compound whether polymeric or otherwise, unless stated to the contrary. In contrast, the term, "consisting essentially of" excludes from the scope of any succeeding recitation any other component, step or procedure, excepting those that are not essential to operability. The term "consisting of" excludes any component, step or procedure not specifically delineated or listed. The term "or", unless stated otherwise, refers to the listed members individually as well as in any combination.

As used with respect to a chemical compound, unless specifically indicated otherwise, the singular includes all isomeric forms and vice versa (for example, "hexane", includes all isomers of hexane individually or collectively). The terms "compound" and "complex" are used interchangeably to refer to organic-, inorganic and organometal compounds. The term, "atom" refers to the smallest constituent of an element regardless of ionic state, that is, whether or not the same bears a charge or partial charge or is bonded to another atom. The term "heteroatom" refers to an atom other than carbon or hydrogen.

"Reaction mixture", "reaction mass" and like terms means the combination of materials necessary or ancillary to a reaction, typically under reactive conditions. Over the course of a reaction, a reaction mixture converts into a product mixture. Depending upon the moment in time tin which the reaction mixture is characterized and other factors such as whether the process is batch or continuous, the physical state of the starting and product materials, etc., it will or can contain the reactants, catalyst, solvent, processing aids, products, byproducts, impurities and the like.

"Product mixture" and like terms means the combination of materials resulting from subjecting a reaction mixture to reaction conditions. A product mixture will always contain some product and/or byproduct and depending upon a multiplicity of factors (e.g., batch versus continuous, physical state of the starting materials, etc.), it may or may not contain unreacted starting materials, catalyst, solvent, processing aids, impurities, and the like. The typical product mixture that forms a part of this invention after the reaction has begun, typically at the end or near the end of the reaction, will include unreacted (meth)acrylic acid and alcohol, strong acid catalyst, (meth)acrylates, byproduct (meth)acrylates and water.

"Reaction conditions" and like terms generally refer to temperature, pressure, reactant concentrations, catalyst concentration, cocatalyst concentration, monomer conversion, product and by-product (or solids) content of the reaction mixture (or mass) and/or other conditions that influence the properties of the resulting product.

"Esterification conditions" and like terms means the temperature, pressure, reactant concentrations, catalyst concentration, cocatalyst concentration, monomer conversion, product and by-product (or solids) content of the reaction mixture (or mass) and/or other conditions necessary to convert (meth)acrylic acid and alcohol into (meth)acrylate ester.

"Hydrolysis conditions" and like terms mean the temperature, pressure, reactant concentrations, catalyst concentration and the like necessary to cleave a compound by reacting it with water, e.g., cleaving a Michael adduct of a (meth)acrylate ester and MSA into the ester and MSA.

"Distillation conditions" and like terms mean the temperature, pressure, compound concentrations, azeotropic agents (if any) and the like necessary to separate at least one compound from one or more other compounds through the action of heating and reflux.

"Continuous process" and like terms means that the process is operated at a steady state, i.e., the reactants are fed to the reactor or reaction zone at a rate substantially in balance with the rate that product is removed from the reactor or reaction zone such that the reaction mass in the reactor or reaction zone is relatively constant in volume and composition. Continuous process does not include a batch or semi-batch process, the former characterized by a depletion of reactants and a growth of product over time, and the latter typically characterized by the unbalanced addition of reactant and removal of product over time.

"(Meth)acrylic acid" means acrylic acid and/or methacrylic acid. "Acrylate" means a salt or ester of acrylic acid, "methacrylate" means a salt or ester of methacrylic acid, and "(meth)acrylate" means a salt or ester of either acrylic acid or methacrylic acid.

"Acrylate reactor blowdown" means the bottom stream or heavy ends from the distillation of the reaction mixture.

"Noble product" and like terms means (meth)acrylic acid monomer, (meth)acrylate monomers, and C₁₋₈ alkanols.

"(Meth)acrylate heavy ends" and like terms means the (meth)acrylate adduct by-products that have a higher boiling point than the principal or desired (meth)acrylate ester product. These heavy ends typically comprise one or more polymeric (meth)acrylates and one or more oligomeric (meth)acrylates. In the context of the present invention, (meth)acrylate heavy ends are typically recovered from the blowdown stream. Specific examples of (meth)acrylate heavy ends are provided in USP 5,877,345.

The (meth)acrylic acid used in the practice, of this invention is either or both acrylic acid or methacrylic acid. Preferably the acid is of at least commercial grade, but crude (meth)acrylic acid can also be used in the practice of the invention. As described in USP 6,472,554, crude (meth)acrylic acid contains as impurities up to 5 weight percent (wt%) acetic acid and up to 1 wt% maleic acid or anhydride.

Any alcohol selected from aliphatic, alicyclic and aromatic alcohols can be used as the alcohol having 1 or more, preferably 4 or more, carbon atoms. Examples of the aliphatic alcohols include methyl alcohol ethyl alcohol, propyl and isopropyl alcohol, butyl alcohol, pentyl alcohol, hexyl alcohol, heptyl alcohol, octyl alcohol, 2-ethylhexyl alcohol, nonyl alcohol, decyl alcohols, dodecyl alcohol, hexadecyl alcohol and stearlyl alcohol. Examples of the alicyclic alcohols include cyclopentyl alcohol, cyclohexyl alcohol, methylcyclohexyl alcohol, ethylcyclohexyl alcohol and butylcyclohexyl alcohol. Examples of the aromatic alcohols include benzyl alcohol, methylbenzyl alcohol, dimethylbenzyl alcohol and butylbenzyl alcohol.

As the acid catalyst for esterification, toluenesulfonic acid, benzenesulfonic acid, xylenesulfonic acid, ethanesulfonic acid, trifluorosulfonic acid and methanesulfonic acid (MSA) are exemplary. MESA is a preferred acid catalyst.

In the esterification reaction, the stalling acrylic or methacrylic acid and alcohol are usually supplied to a reactor in a molar ratio of 1.0:1.2-1.0:0.8. The amount of the acid catalyst used is generally from 0.1 to 5, preferably from 0.5 to 2.0, percent by weight of the reactants. The reaction is conducted generally at a temperature of 70 to 180°C. Reaction water produced in the course of the esterification reaction is preferably removed by distillation or azeotropic distillation (reaction-distillation method). In order to facilitate the removal of reaction water, an inert azeotropic agent may be used. Hydrocarbons such as benzene, toluene and cyclohexane are exemplary azeotropic agents. The removal of reaction water may be conducted by other means as well, such as membrane separation using a vapor separation membrane, or a per-evaporation membrane, or by a method other than distillation. In order to prevent the loss of acrylic or methacrylic acid or the ester due to the occurrence of unfavorable polymerization, a polymerization inhibitor, e.g., phenothiazine, hydroquinone, the methyl ester of hydroquinone, a TEMPO derivative or an oxygen-containing gas, is usually added to the reactor. The (meth)acrylate ester and by products are simultaneously recovered with the reaction water by distillation or azeotropic distillation, and then purified.

The residue from the reaction vessel comprises mainly low molecular weight polymers of (meth)acrylate acid and (meth)acrylate ester, some free (meth)acrylic acid, alcohol and catalyst and it is subjected to heat treatment (cracking). While most of the heat-treated material is returned to the reactor, a slip stream of heavy ends is sent to a heavy ends removal unit, e.g., distillation column. The volatiles from the heavy ends removal unit are recycled to the reactor while the residue is discarded unless subjected to further treatment.

In another embodiment, e.g., such as that taught in USP 5,386,052, only the water of reaction is removed. The reaction liquid, e.g., the product mixture, is discharged from the reactor after the esterification reaction is completed.

The reaction liquid can be cooled to a temperature of 10 to 60°C, and then washed with water for extraction. The temperature of the wash water is preferably the same as or somewhat lower than the temperature of the reaction liquid. The weight ratio of the wash water to the reaction liquid is preferably 0.5 or less, more preferably from 0.05 to 0.2. Although fresh water can be used as the wash water, the reaction water produced in the esterification reaction and removed from the reaction system may also be employed. The use of the latter has the advantage that the amount of waste water can be reduced.

The washing with water can be conducted in various manners. For example, water and the reaction liquid are mixed under agitation, and then the mixture is allowed to stand to separate into aqueous and organic phases. Alternatively, the washing and the liquid-liquid separation are conducted by means of a centrifuge. Most effectively, the washing is conducted by means of an extraction column in which the liquid-liquid contact is made with the application of a weak stirring force, so that little, if any, emulsion is formed and thus the liquid-liquid separation is readily made.

Any extraction column can be used in the practice of this invention. Typically, the reaction liquid is fed into the extraction column at its lower end, and water into its upper end. The reaction liquid from which the catalyst and acrylic or methacrylic acid are removed is obtained from the top of the column, and an aqueous solution containing the catalyst, acrylic or methacrylic acid, and by-products is removed from the bottom of the column. A packed column, a tray tower or the like is usually used as an extraction column.

Catalyst and part of the (meth)acrylic acid are removed as an aqueous stream from the bottom of the column, and the remainder of the (meth)acrylic acid, (meth)acrylate product alcohol are removed as an organic stream from the top of the column. This overhead stream is further washed, either with water or an aqueous alkaline solution to further remove the remaining catalyst and acid. The aqueous acid stream can be returned to the reactor, and the washed overhead stream is then subjected to a first distillation to recover the principal product as a first distillation overhead stream. The remainder of the washed overhead stream is recovered as a first distillation bottom stream containing the heavy ends (i.e., the oligomers and polymers of the (meth)acrylate esters), catalyst, processing aids, etc.

In one practice, this first distillation bottom stream is simply discarded in any acceptable manner, e.g., incineration, and its values lost. In another practice, this first distillation bottom stream is first subjected to another water washing, e.g., mixed with an equal volume of water, and then allowed to separate. The aqueous phase contains additional recovered sulfonic acid and unreacted starting material, and the heavy ends is discarded in any acceptable manner with the concomitant loss of acrylate and catalyst values contained in the oligomers and polymers of the (meth)acrylate esters. In still another practice, the heavy ends are subjected to cracking and/or hydrolysis, and the then subjected to another water wash for the recovery of the acrylate and sulfonic acid values.

According to this invention, however, this first distillation bottom stream or the bottom stream from the direct distillation of the reaction mixture is subjected to a second distillation in which the oligomers and polymers are cracked and/or hydrolyzed, and nobles products and catalyst are recovered. This second distillation results in an increased recovery or the sulfonic acid catalyst by cracking/hydrolysis of the bound acid, the Michael adduct of the acid and acrylate ester, and simultaneous recovery of noble products.

The first distillation bottom stream, or acrylate heavy ends, is first mixed with water or an aqueous stream from any convenient source, e.g., recovered process water. Topically, equal volumes of water and heavy ends are mixed with one another, although the weight ratio of water to heavy ends can vary widely, e.g., between 10:1 and 1:10, preferably between 5:1 and 1:5 and more preferably between 1:1 and 1:2. The mixture is then subjected to conditions sufficient to crack and/or hydrolyze the Michael adduces present in the heavy ends into their constituent parts of (meth)acrylate ester, (meth)acrylic acid, alcohol and sulfonic acid. These conditions typically include agitation, e.g., stirring, turbulent flow, etc., and a temperature between 100 and 150, preferably between 100 and 120 and more preferably between 100 and 110, °C. The cracking/hydrolysis with simultaneous distillation is typically conducted in a distillation column of any design, and proceeds until the organic phase no longer appears, or appears in a substantially reduced amount, in the overhead, e.g., for a period of 1 to 24, preferably 2 to 12 and more preferably 4 to 8, hours. The process can be conducted in a batch, semi-batch or continuous mode. The water/heavy ends mixture can optionally contain an azeotropic agent, and the amount of agent present in the acrylate heavy ends is typically sufficient for the process. The water phase of the residue after the cracking/hydrolysis distillation is complete, can be separated and recycled to the reactor.

This recovery process is exemplified by the following examples.

### SPECIFIC EMBODIMENTS

### Comparative Example 1:

Seventy-live grams each of butyl acrylate reactor blowdown (after recovering acrylic acid, butanol, and butyl acrylate) containing 3.21 wt% MSA by titration and 5.61 wt% by sulfur and nitrogen analyses (nitrogen analysis is used to exclude phenothiazine contribution to the sulfur content) is mixed with aqueous feed for 15 minutes. The phases are allowed to settle for 45 minutes and then separate. The organic phase is 54 wt% of the total mass and has 0.10 wt% MSA by titration and 1.45 wt% by sulfur and nitrogen analyses. The aqueous and rag layer, i.e., the interfacial layer between the aqueous and organic layers, combined is 46% of the total mass and had 3.15 wt% MSA by titration and 4.20 wt% by sulfur and nitrogen analyses. The calculated MSA recovery by titration is 90 wit%. The calculated MSA recovery by sulfur and nitrogen analyses is only 71 wt%. (Table 1A).

### Comparative Example 2:

Seventy-five grams each of butyl acrylate reactor blowdown (after recovering acrylic acid, butanol, and butyl acrylate) containing 3.13 wt% MSA by titration and 4.94 wt% by sulfur and nitrogen analyses (nitrogen analysis is used to exclude phenothiazine contribution to the sulfur content) is mixed with aqueous feed for 15 minutes. The phases are allowed to settle for 45 minutes and then separate. The organic phase is 49 wt% of the total mass and had 0.10 wt% MSA by titration and 1.47 wt% by sulfur and nitrogen analyses. The aqueous and rag layer combined is 51 wt% of the total mass and had 2.89 wt% MSA by titration and 3.90 wt% by sulfur and nitrogen analyses. The calculated MSA recovery by titration is 94 wit%. The calculated MSA recovery by sulfur and nitrogen analyses is only 74%. (Table 1A).

**Table 1A**

| Comparison of MSA Recovery by Titration and Sulfur Analyses | | | | | | | |
|---|---|---|---|---|---|---|---|
| | MSA (wt%) | N Conc. (wt%) | S Conc. (wt%) | Wt. (g) | Calc. PTZ (wt%) | MSA By N&S Anal. (wt%) | Titrated MSA to Total MSA (%) |
| Comp. Ex. 1 | | | | | | | |
| Aqueous Feed | 0 | 0 | 0 | 74.77 | 0 | 0 | NA |
| Bbowdown | 3.21 | 0.275 | 2.5 | 74.46 | 3.909 | 5.614 | 57 |
| | | | | | | | |
| Aqueous Phase | 3.15 | 0 | 1.4 | 68.52 | 0 | 4.2 | 75 |
| Organic Phase | 0.1 | 0.27 | 1.1 | 79.44 | 3.838 | 1.449 | 7 |
| | | | | | | | |
| Accountability(%) | | | | 99 | 105 | 96 | |
| MSA Recovery | 90 | | | | | 71 | |
| | | | | | | | |

| Comp. Ex. 2 | | | | | | | |
|---|---|---|---|---|---|---|---|
| Aqueous Feed | 0 | 0 | 0 | 74.8 | 0 | 0 | NA |
| Blowdown | 3.13 | 0.395 | 2.55 | 74.37 | 5.615 | 4.941 | 63 |
| | | | | | | | |
| Aqueous Phase | 2.89 | 0 | 1.3 | 75.98 | 0 | 3.9 | 74 |
| Organic Phase | 0.1 | 0.355 | 1.3 | 72.73 | 5.046 | 1.466 | 7 |
| | | | | | | | |
| Accountability (%) | | | | 100 | 88 | 110 | |
| MSA Recovery | 94 | | | | | 74 | |

### Example 1:

Butyl acrylate reactor blowdown (161.35g and containing 4.2 wt% acrylic acid, 0.17 wt% butanol, and 6.5 wt% butyl acrylate by gas chromatograph (GC) and 2.02 wt% MSA by titration) and 148.25g of aqueous feed are placed in a round bottom flask equipped with a stirrer, a condenser and a distillate receiver. The mixture is distilled while kept under agitation at 100-104°C and atmospheric pressure for five hours. During the distillation the aqueous distillate is continually returned to the distillation flask. The distillation flask mixture is then allowed to cool and the phases are separated after 45 minutes of settling time. 50.14g of organic containing 4.9 wt% acrylic acid, 36.1 wt% butanol and 20.8 wt°%: butyl acrylate is collected as distillate. The aqueous phase from the distillation flask is 184.93g and contains 4.6 wt% acrylic acid, 0.86 wt% butanol and 2.33 wt% MSA by titration. The remaining organic phase from the distillation flask is 69.26g and contains 0.21 wt% MSA by titration. The titration-based MSA recovery is 132 wt% and acrylic acid, butanol, and butyl acrylate recovery is 7, 12 and 6 wt%, respectively. (Table 1B).

### Example 2:

Butyl acrylate reactor blowdown (1557.74g and containing 9.8 wt% acrylic acid, 0.13 wt% butanol, and 6.9 wt% butyl acrylate by GC and 2.4 wt% MSA by titration) and 147.98g of aqueous feed are placed in a round bottom flask equipped with a stirrer, a condenser and a distillate receiver. The mixture is distilled while kept under agitation at 100-101°C and atmospheric pressure for seven and one-half hours. During the distillation the aqueous distillate is continually returned to the distillation flask, Th e distillation flask mixture is then allowed to cool and the phases are separated after 45 minutes of settling time. Organic (48.49g) containing 8.6 wt% acrylic acid, 45 wt% butanol and 24.4 wt% butyl acrylate is collected as distillate. The aqueous phase from the distillation flask is 200.58g and contains 7.7 wt% acrylic acid, 0.59 wt% butanol and 2.71 wt% MSA by titration. The remaining organic phase from the distillation flask is 53.09g and contains 0.23 wt% MSA by titration. The titration-based MSA recovery is 144 wt% and acrylic acid, butanol, and butyl acrylate recovery was 12,15 and 8 wt%, respectively. (Table 1B).

### Example 3:

Butyl acrylate reactor blowdown (162.18g and containing 3.2 wt% acrylic acid, 0.05 wt% butanol, and 2.5 wt% butyl acrylate by GC and 2.71 wt% MSA by titration) and 148.63g of aqueous feed are placed in a round bottom flask equipped with a stirred, a condenser and a distillate receiver. The mixture is distilled while kept under agitation at 100-103°C and atmospheric pressure for five hours. During the distillation the aqueous distillate is continually return to the distillation flask. The distillation flask mixture is then allowed to cool and the phases are separated after 45 minutes of settling time. Organic (45.72g) containing 12.1 wt% acrylic acid, 45.5 wt% butanol and 11.8 wt% butyl acrylate is collected as distillate. The aqueous phase from the distillation flask is 201.96g and contains 11.6 wt% acrylic acid, 0.83 wt% butanol and 2.86 wt% MSA by titration. The remaining organic phase from the distillation flask is 57.47g and contains 0.39 wt% MSA by titration. The titration-based MSA recovery is 131 wt% and acrylic acid, butanol, and butyl acrylate recovery was 18,14 and 3 wt%, respectively. (Table 1B).

### Example 4:

Butyl acrylate reactor blowdown (161.32g and containing 11.4 wt% acrylic acid, 0.09 wt% butanol, and 6.5 wt% butyl acrylate by GC and 2.53 wt% MSA by titration) and 147.78g of aqueous feed are placed in a round bottom flask equipped with a stirrer, a condenser and a distillate receiver. The mixture is distilled while kept under agitation at 100-102°C and atmospheric pressure for four and one-half hours. During the distillation the aqueous distillate is continually returned to the distillation flask. The distillation flask mixture is then allowed to cool and the phases are separated after 45 minutes of settling time. Organic (48.73g) containing 9.6 wt% acrylic acid, 43.6 wt% butanol and 30.6 wt% butyl acrylate is collected as distillate. The aqueous phase from the distillation flask is 199.33g and contains 7.1 wt% acrylic acid, 1.12 wt% butanol and 2.92 wt% MSA by titration. The remaining organic phase from the distillation flask is 57.36g and contains 0.43 wt% MSA by titration. The titration-based MSA recovery is 143 wt% and acrylic acid, butanol, and butyl acrylate recovery was 12, 15 and 9 wt%, respectively. (Table 1B).

**Table 1B**

| Distillation of Extraction Mixture at 1:1 Water to Organic Feed Volume Ratio | | | | |
|---|---|---|---|---|
| | Example 1 | Example 2 | Example 3 | Example 4 |
| Distillation Time (hr) | 5 | 7.5 | 5 | 4.5 |
| Temperature Range (°C) | 102 | 100-101 | 100-103 | 100-102 |
| Weight Ratio of Aqueous to Organic Feed at Distillation | 0.92 | 0.94 | 0.92 | 0.92 |
| | | | | |
| Aqueous Feed (g): | 148.25 | 147.98 | 48.63 | 147.78 |
| Butyl Acrylate (%) | 0.08 | 0.1 | 0.1 | 0.1 |
| Butanol(%) | 2.76 | 2.6 | 2.62 | 2.6 |
| Acrylic Acid (%) | 0 | 0 | 0 | 0 |
| Water (%) | 93.56 | 92.25 | 98.27 | 92.25 |
| MSA (%) | 0 | 0 | 0 | 0 |
| Unknowns (%) | 3.6 | 5.05 | 0 | 5.05 |
| | | | | |
| Blowdown Feed (g): | 161.35 | 157.74 | 162.18 | 161.32 |
| Butyl Acrylate (%) | 6.48 | 6.86 | 2.53 | 6.48 |
| Butanol (%) | 0.17 | 0.13 | 0.05 | 0.09 |
| Acrylic Acid (%) | 4.2 | 9.8 | 3.2 | 11.4 |
| Water (%) | 0 | 0 | 0 | 0 |
| MSA(%) | 2.02 | 2.4 | 2.71 | 2.53 |
| Unknowns(%) | 87.2 | 80.8 | 91.5 | 79.6 |
| | | | | |
| Organic Distillate(g); | 50.14 | 48.49 | 45.72 | 48.73 |
| Blutyl Acrylate (%) | 20.82 | 24.42 | 11.82 | 30.56 |
| Butanol (%) | 36.08 | 45.03 | 45.5 | 43.56 |
| Acrylc Acid (%) | 4.9 | 8.6 | 12.1 | 9.6 |
| Water (%) | 8.17 | 10.07 | 12.09 | 9.53 |
| MSA (%) | 0 | 0 | 0 | 0 |
| Unknowns (%) | 30.07 | 11.85 | 18.47 | 6.76 |
| | | | | |
| Aqueous Extract (g): | 184.93 | 200.58 | 201.96 | 199.33 |
| Butyl Acrylate (%) | 0 | 0 | 0 | 0.03 |
| Butanol (%) | 0.86 | 0.59 | 0.83 | 1.12 |
| Acrylic Acid (%) | 4.6 | 7.7 | 11.6 | 71.1 |
| Water (%) | 75.69 | 3.59 | 65.21 | 64.9 |
| MSA (%) | 2.33 | 2.71 | 2.86 | 2.92 |
| Unknowns (%) | 16.53 | 15.431 | 19.48 | 23.91 |
| | | | | |
| Organic Raffinate (g): | 69.26 | 53.09 | 57.47 | 57.36 |
| Butyl Acrylate (%) | 0.08 | 0.22 | 0.13 | 0.54 |
| Butanol (%) | 2.30 | 1.38 | 1.79 | 2.58 |
| Acrylic Acid (%) | 7.1 | 12.3 | 16 | 10.6 |
| Water (%) | 3.84 | 6.19 | 5.45 | 6.56 |
| MSA (%) | 0.21 | 0.23 | 0.39 | 0.43 |
| Unknowns (%) | 86.44 | 79.68 | 76.28 | 79.25 |
| | | | | |
| Calculation: | | | | |
| Accountability (%) | 98 | 99 | 98 | 99 |
| MSA (Titration) | 132 | 144 | 131 | 143 |
| Butyl Acrylate (%) | 6 | 8 | 3 | 9 |
| Butanol (%) | 12 | 15 | 14 | 15 |
| Acrylic Acid (%) | 7 | 12 | 18 | 12 |

## Claims

1. A process for the recovery of a sulfonic acid and noble products from (nieth)acrylate heavy ends, the heavy ends comprising the sulfonic acid, noble products and Michael adducts of the sulfonic acid and (meth)acrylate esters, the process comprising the steps of (i) mixing the heavy ends with water, (ii) subjecting the mixture to sufficient heat and distillation conditions to crack and/or hydrolyze the Michael adducts into its constituent sulfonic acid and acrylate ester components, and (iii) recovering the noble products as a distillate stream and the sulfonic acid as an aqueous phase.

2. The process of Claim 1 in which the cracking and distillation conditions include agitation of the mixture.

3. The process of Claim 1 in which the cracking and distillation conditions include a temperature in the range of 100 to 150°C.

4. The process of Claim 1 in which the acrylate heavy ends arc mixed with an equal volume of water.

5. The process of Claim 1 in which the noble products include at least one (meth)acrylic acid monomer, (meth)acrylate monomer, and C₁₋₈ alkanol.

6. The process of Claim 1 in which the noble product includes acrylic acid, butyl alcohol and butyl acrylate.

7. The process of Claim 1 in which the acrylate heavy ends comprise an azeotropic agent.

8. The process of Claim 1 in which the sulfonic acid is at least one of toluenesulfonic acid, benzenesulfonic acid, xylenesulfonic acid, ethanesulfonic acid, trifluorosulfonic acid and methanesulfonic acid.

9. The process of Claim 1 in which the acrylate heavy ends comprise oligomers and polymers of butyl acrylate.

10. The process of Claim 1 in which the acrylate heavy ends comprise Michael adducts of methanesulfonic acid and butyl acrylate.

11. The process of Claim 7 in which the azeotropic agent is at least one of benzene, toluene and cyclohexane.

12. A process for producing a (meth)acrylate ester, the process comprising the steps of:
A. Subjecting a reaction mixture comprising (meth)acrylic acid, an alcohol and a sulfonic acid catalyst to esterification conditions so as to form a product mixture comprising unreacted (meth)acrylic acid and alcohol, sulfonic acid catalyst, (meth)acrylate ester, and oligomeric and polymeric (meth)acrylate esters;
B. Distilling the product mixture to recover (meth)acrylate ester, and to leave a distillation residue comprising sulfonic acid catalyst and oligomeric and polymeric acrylate esters;
C. Mixing the distillation residue with water to form a mixture of distillation residue and water;
D. Subjecting the distillation residue and water mixture of (C) to conditions such that, simultaneously, (1) oligomeric and polymeric (meth)acrylate esters are hydrolyzed and/or cracked to (meth)acrylate ester, (meth)acrylic acid, alcohol and sulfonic acid, (2) (meth)acrylic acid, alcohol and (meth)acrylate ester are recovered as an overhead distillation product, and (3) sulfonic acid catalyst is recovered by aqueous phase separation of the distillation residue and water mixture as a bottom product; and
E. Recycling at least a part of the overhead distillation and the bottom products of (D) to the reaction mixture of (A).

13. The process of Claim 12 in which the alcohol is a C₁₋₈ alkanol.

14. The process of Claim 13 in which the sulfonic acid is at least one of toluenesulfonic acid, benzenesulfonic acid, xylenesulfonic acid, ethanesulfonic acid, trifluorosulfonic acid and methanesulfonic acid.

15. The process of Claim 14 in which the distillation residue and water of step (C) are mixed at a weight ratio of water to residue of 5:1 and 1:5.

16. The process of Claim 15 in which the oligomeric and polymeric (meth)acrylate esters are hydrolyzed and/or cracked at a temperature in the range of the 100 to 150°C.

## Patentansprüche

1. Verfahren zur Rückgewinnung von Sulfonsäure und hochwertigen Produkten aus hochsiedenden (Meth)acrylatfraktionen, wobei die hochsiedenden Fraktionen die Sulfonsäure, die hochwertigen Produkte und Michael-Addukte aus Sulfonsäure und (Meth)acrylatestern enthalten und wobei das Verfahren die folgenden Schritte umfasst: (i) die hochsiedenden Fraktionen werden mit Wasser gemischt, (ii) das Gemisch wird Wärme- und Destillationsbedingungen ausgesetzt, die ausreichend sind, um die Michael-Addukte in ihre Bestandteile Sulfonsäure und Acrylatesterkomponente zu spalten und/ oder zu hydrolysieren, und (iii) die hochwertigen Produkte werden als Destillatstrom und die Sulfonsäure wird als wässrige Phase gewonnen.

2. Verfahren nach Anspruch 1, wobei die Spalt- und Destillationsbedingungen das Rühren des Gemisches umfassen.

3. Verfahren nach Anspruch 1, wobei die Spalt- und Destillationsbedingungen eine Temperatur im Bereich von 100 bis 150 °C umfassen.

4. Verfahren nach Anspruch 1, wobei die hochsiedende Acrylatfraktion mit dem gleichen Volumen Wasser vermischt wird.

5. Verfahren nach Anspruch 1, wobei die hochwertigen Produkte zumindest (Meth)acrylsäuremonomer, (Meth)acrylatmonomer und C₁₋₈-Alkanol umfassen.

6. Verfahren nach Anspruch 1, wobei das hochwertige Produkt Acrylsäure, Butylalkohol und Butylacrylat umfasst.

7. Verfahren nach Anspruch 1, wobei die hochsiedende Acrylatfraktion einen azeotropen Stoff enthält.

8. Verfahren nach Anspruch 1, wobei es sich bei der Sulfonsäure um zumindest eine der folgenden Säuren handelt:
Toluolsulfonsäure, Benzolsulfonsäure, Xylolsulfonsäure, Ethansulfonsäure, Trifluorsulfonsäure und Methansulfonsäure.

9. Verfahren nach Anspruch 1, wobei die hochsiedende Acrylatfraktion Oligomere und Polymere von Butylacrylat enthält.

10. Verfahren nach Anspruch 1, wobei die hochsiedende Acrylatfraktion Michael-Addukte von Methansulfonsäure und Butylacrylat enthält.

11. Verfahren nach Anspruch 7, wobei es sich bei dem azeotopen Stoff um zumindest einen aus der Gruppe Benzol, Toluol und Cyclohexan handelt.

12. Verfahren zur Herstellung eines (Meth)acrylatesters, das die folgenden Schritte umfasst:
A. ein Reaktionsgemisch, das (Meth)acrylsäure, einen Alkohol und einen Sulfonsäurekatalysator enthält, wird zur Bildung eines Produktgemisches, das nicht umgesetzte (Meth)acrylsäure und nicht umgesetzten Alkohol, Sulfonsäurekatalysator, (Meth)acrylsäureester und oligomere und polymere (Meth)acrylsäureester enthält, Veresterungsbedingungen ausgesetzt;
B. das Produktgemisch wird destilliert, um (Meth)acrylsäureester zu gewinnen und einen Destillationsrückstand zu erhalten, der Sulfonsäurekatalysator und oligomere und polymere Acrylatester enthält;
C. der Destillationsrückstand wird mit Wasser vermischt, um ein Gemisch aus Destillationsrückstand und Wasser zu bilden;
D. das Gemisch aus Destillationsrückstand und Wasser aus (C) wird solchen Bedingungen ausgesetzt, dass gleichzeitig (1) die oligomeren und polymeren (Meth)acrylatester in (Meth)acrylatester, (Meth)acrylsäure; Alkohol und Sulfonsäure hydrolysiert und/oder gespalten werden, (2) (Meth)acrylsäure, Alkohol und (Meth)acrylatester als Kopfdestillationsprodukt gewonnen werden und (3) der Sulfonsäurekatalysator durch Wässrigphasentrennung des Gemisches aus Destillationsrückstand und Wasser als Sumpfprodukt gewonnen wird; und
E. zumindest ein Teil des Kopfdestillationsprodukts und des Sumpfprodukts aus (D) zu dem Reaktionsgemisch (A) rückgeführt wird.

13. Verfahren nach Anspruch 12, wobei der Alkohol ein C₁₋₈-Alkanol ist.

14. Verfahren nach Anspruch 13, wobei es sich bei der Sulfonsäure um zumindest eine der folgenden Säuren handelt:
Toluolsulfonsäure, Benzolsulfonsäure, Xylolsulfonsäure, Ethansulfonsäure, Trifluorsulfonsäure und Methansulfonsäure.

15. Verfahren nach Anspruch 14, wobei der Destillationsrückstand und das Wasser in Schritt (C) in einem Gewichtsverhältnis von 5 : 1 bis 1 : 5 gemischt werden.

16. Verfahren nach Anspruch 15, wobei die oligomeren und polymeren (Meth)acrylatester bei einer Temperatur im Bereich von 100 bis 150 °C hydrolysiert und/oder gespalten werden.

## Revendications

1. Procédé de récupération d'un acide sulfonique et de produits nobles à partir de fractions lourdes (méth)acrylate, les fractions lourdes comprenant l'acide sulfonique, les produits nobles et des adduits de Michael de l'acide sulfonique et d'esters (méth)acrylate, le procédé comprenant les étapes de
(i) mélange des fractions lourdes avec de l'eau,
(ii) soumission du mélange à une chaleur suffisante et à des conditions de distillation pour craquer et/ou hydrolyser les adduits de Michael en leurs composants constitutifs acide sulfonique et ester acrylate, et
(iii) la récupération des produits nobles sous la forme d'un courant de distillat et de l'acide sulfonique sous la forme d'une phase aqueuse.

2. Procédé selon la revendication 1, dans lequel les conditions de craquage et de distillation comprennent l'agitation du mélange.

3. Procédé selon la revendication 1, dans lequel les conditions de craquage et de distillation comprennent une température dans la plage allant de 100 à 150 °C.

4. Procédé selon la revendication 1, dans lequel les fractions lourdes acrylate sont mélangées avec un volume égal d'eau.

5. Procédé selon la revendication 1, dans lequel les produits nobles comprennent au moins un monomère acide (méth) acrylique, un monomère (méth)acrylate et un alcanol en C₁₋₈.

6. Procédé selon la revendication 1, dans lequel les produits nobles comprennent de l'acide acrylique, de l'alcool butylique et de l'acrylate de butyle.

7. Procédé selon la revendication 1, dans lequel les fractions lourdes acrylate comprennent un agent azéotropique.

8. Procédé selon la revendication 1, dans lequel l'acide sulfonique est l'acide toluène sulfonique et/ou l'acide benzène sulfonique et/ou l'acide xylène sulfonique et/ou l'acide éthane sulfonique et/ou l'acide trifluorosulfonique et/ou l'acide méthane sulfonique.

9. Procédé selon la revendication 1, dans lequel les fractions lourdes acrylate comprennent des oligomères et des polymères d'acrylate de butyle.

10. Procédé selon la revendication 1, dans lequel les fractions lourdes acrylate comprennent des adduits de Michael d'acide méthane sulfonique et d'acrylate de butyle.

11. Procédé selon la revendication 7, dans lequel l'agent azéotropique est le benzène et/ou le toluène et/ou le cyclohexane.

12. Procédé de fabrication d'un ester (mëth)acrylate, le procédé comprenant les étapes suivantes :
A. la soumission d'un mélange réactionnel comprenant de l'acide (méth)acrylique, un alcool et un catalyseur acide sulfonique à des conditions d'estérification pour former un mélange de produits comprenant de l'acide (méth)acrylique et de l'alcool non réagis, le catalyseur acide sulfonique, un ester (méth)acrylate et des esters (méth)acrylate oligomères et polymères ;
B. la distillation du mélange de produits pour récupérer l'ester (mëth)acrylate, et pour laisser un résidu de distillation comprenant le catalyseur acide sulfonique et les esters acrylate oligomères et polymères ;
C. le mélange du résidu de distillation avec de l'eau pour former un mélange du résidu de distillation et d'eau ;
D. la soumission du mélange du résidu de distillation et de l'eau de (C) à des conditions telles que, simultanément, (1) les esters (méth)acrylate oligomères et polymères soient hydrolysés et/ou craqués en ester (méth)acrylate, acide (méth)acrylique, alcool et acide sulfonique, (2) l'acide (méth)acrylique, l'alcool et l'ester (méth)acrylate soient récupérés sous la forme d'un produit de distillation de tête, et (3) le catalyseur acide sulfonique soit récupéré par séparation de phase aqueuse du mélange du résidu de distillation et de l'eau en tant que produit de fond ; et
E. le recyclage d'au moins une partie des produits de distillation de tête et de fond de (D) dans le mélange réactionnel de (A).

13. Procédé selon la revendication 12, dans lequel l'alcool est un alcanol en C₁₋₈.

14. Procédé selon la revendication 13, dans lequel l'acide sulfonique est l'acide toluène sulfonique et/ou l'acide benzène sulfonique et/ou l'acide xylène sulfonique et/ou l'acide éthane sulfonique et/ou l'acide trifluorosulfonique et/ou l'acide méthane sulfonique.

15. Procédé selon la revendication 14, dans lequel le résidu de distillation et l'eau de l'étape (C) sont mélangés en un rapport en poids entre l'eau et le résidu de 5:1 à 1:5.

16. Procédé selon la revendication 15, dans lequel les esters (méth)acrylate oligomères et polymères sont hydrolysés et/ou craqués à une température dans la plage allant de 100 à 150°C.
